# EUROPEAN PATENT SPECIFICATION

(11) **EP 0 421 557 B1**
(45) Date of publication and mention of the grant of the patent: **29.09.1993**
(21) Application number: 90202828.1
(22) Date of filing: 23.07.1984
(51) Int. Cl.: A61M 1/00, H02K 37/00, A61F 2/00

(54) **Method and apparatus for indirect and external adjustment of the setting pressure of a shunt valve**
Verfahren und Einrichtung zur indirekten und externen Regelung des Betriebdruckes eines Parallelventils
Procédé et dispositif pour l'ajustement indirect et externe de la pression de fonctionnement d'une soupape de derivation

(30) Priority: 21.07.1983 US 516137; 08.12.1983 US 559864; 08.12.1983 US 559865
(43) Date of publication of application: 10.04.1991
(62) Divisional of application: 84304995.8
(73) Proprietor: Hakim, Salomon, Bogota (CO); Hakim, Carlos A., Fort Lauderdale Florida 33308 (US)
(72) Inventor: Hakim, Salomon, Bogota (CO); Hakim, Carlos A., Fort Lauderdale Florida 33308 (US)
(74) Representative: Deans, Michael John Percy

(56) References cited:
- EP-A- 0 060 369
- DE-B- 2 626 215
- US-A- 4 360 007

## Description

This invention relates to a method and apparatus for indirect and external adjustment of the popping pressure of a shunt valve.

We describe below surgically implantable shunt valves for venting cerebrospinal fluid ("CSF") in the treatment of hydrocephalus and similar conditions;of impaired circulation of absorption of body fluids.

Cerebrospinal fluid shunt valves have been in use for over twenty years. Broadly speaking, they function by venting excess cerebrospinal fluid from the brain into the venous system or other receptive cavities (e.g., peritoneal, pleural). Many such valves, including the earliest designs, operate by controlling the amount of fluid flow The neurosurgeon makes an estimate of the amount of flow required to relieve the hydrocephalus and selects a valve of that flow capacity. The selection is made difficult by the wide variation in normal flow rates.

About twenty years ago, one of the present applicants, namely Salomon Hakim, developed an altogether different valve, one that controlled intraventricular pressure rather than flow. That valve, which is today known as the Cordis-Hakim shunt valve, and which is described in U.S. Patent No. 3,288,142 has been enormously successful and remains, even today, one of the most popular shunt valves in use. It has a spherical sapphire ball biased against a conical valve seat by a stainless steeil spring. The pressure of cerebrospinal fluid pushes against the sapphire ball and spring in a direction tending to raise the ball from the seat. When the pressure difference across the valve (e.g., the pressure difference between the cerebral ventricle and the drainage site) exceeds a so-called popping pressure, the ball rises from the seat to vent cerebrospinal fluid. As the flow rate through the valve increases, the ball moves further away from the seat to provide a larger valve orifice, one that is always large enough that the pressure drop across the orifice never rises much above the popping pressure. Accordingly, the differential pressure across the valve remains nearly constant for any flow rate encountered within the cerebrospinal fluid system.

As successful as the Cordis-Hakim valve has been, it has one important limitation. It can only provide a fixed popping pressure. In treating hydrocephalus, it is often desirable to vary the popping pressure in accordance with ventricle size and treatment objective. For example, initial treatment may require a lower than normal pressure to initiate shrinkage of the ventricles, but as the ventricles decrease in size, the popping pressure should be increased gradually so that when the ventricles return to normal size the intraventricular pressure is at its normal value and the intracranial force systems are in balance (i.e., the popping pressure is set at a level that will stabilize the ventricles at a desired size). Generally speaking, the popping pressure should be varied inversely with the ventricle size. It is undesirable to leave a low pressure valve in a patient after the ventricles are again normal size, because the ventricles can further collapse, leading to a condition known as "slit" ventricles. A fuller discussion of these matters can be found in Hakim et al., "A Critical Analysis of Valve Shunts Used in the Treatment of Hydrocephalus", Developmental Medicine and Child Neurology, Vol. 15, No. 2, April 1973, pp. 230-255.

A further reason for providing adjustablility in popping pressure is to correct for the wide variation in nominal popping pressure typical in manufactured valves. With an adjustable valve, the popping pressure can be more accurately set at the factory, and can be checked, and corrected if necessary, in the operating room prior to implantation. Moreover it is unnecessary to manufacture and stock valves with differing nominal pressures, as one valve can typically provide all desired pressures according to the needs at any given moment of the treatment.

Efforts have been made at developing an adjustable valve. An example is a valve disclosed in our earlier-filed copending United States application Serial No. 493,748, in which an adjustment screw is turned either by a screw driver applied through the skin to the valve or by rotation of a magnet along an axis aligned with the axis of the screw.

Implantable magnetically-driven devices have been proposed Levy et al. U.S. Patent No. 4,360,007 discloses an implantable actuator with a ratchet wheel, pawl, and permanent magnet; application of an external magnetic field rotates the implanted magnet and pawl to advance the ratchet wheel.

Stepping motors have been known per se for many years. The simplest stepping motor consists of a permanent magnet rotor surrounded by a stator made up of four electromagnets. By selectively energizing the electromagnets, it is possible to turn the rotor in angular "steps" of 90°. Often the rotor has a plurality of permanent magnets, so as to reduce the angular step size. Some stepping motors replace the permanent magnets on the rotor with regions of different magnetic reluctance; in these, known as variable reluctance motors, the rotor turns to the position of minimum reluctance. Still others, known as hybrids, combine reluctance differences with permanent magnets.

Stepping motors have been used in some medical applications. For example, they have been used in medical infusion pumps for delivering precise volumetric dosages of drugs at prescribed time intervals. In all these applications the stepping motor was located outside the patient's body, either in a portable device carried by the patient or in a bedside unit.

Other types of medical devices (e.g., pacemakers) have been implanted in the body. These have typically relied for a power source on batteries implanted along with the device.

According to a first aspect of the present invention, we provide a method of indirect external adjustment of the popping pressure of a shunt valve of the type having one or more members adapted to move under the influence of a magnetic field, the method being characterised in comprising the steps of: positioning an adjustment device in proximity to said valve, said adjustment device including a plurality of electromagnets; and selectively energizing said electromagnets through a sequence of steps in which different ones of said electromagnets are energized at successive steps; the sequence of energization and the orientation and strength of said electromagnets being such that said member(s) is (are) caused to move in response to said sequence of energization and to thereby adjust said popping pressure.

In a second and alternative aspect thereof, the invention provides apparatus for externally programming a shunt valve whereby indirectly and externally to adjust its popping pressure, said shunt valve being of the type having one or more members adapted to move under the influence of a magnetic field, said apparatus being characterised in comprising: a housing with a plurality of electromagnets supported on or within said housing; and means electrically connected to said electromagnets and adapted for selectively energizing said electromagnets through a sequence of steps in which different ones of said electromagnets are energized at successive steps, the sequence of energization and the orientation and strength of said electromagnets being selected such that said member(s) in said valve is (are) caused to move in response to said sequence of energization to thereby adjust said popping pressure.

The invention is hereinafter more particularly described by way of example only with reference to the accompanying drawings, in which:-
Fig. 1 is a perspective, somewhat diagrammatic, view of an assembly of shunt valves and catheters for implantation to relieve cerebrospinal fluid pressure;
Fig. 2 is a cross-sectional view taken at 2-2 of Fig. 1, showing the internal construction of one said shunt valve;
Fig. 3 is a cross-sectional view taken at 3-3 in Fig. 2;
Fig. 4 is an elevation view taken at 4-4 in Fig. 2;
Fig. 5 is a plan view at 5-5 in Fig. 2, showing the cam of said embodiment of shunt valve;
Fig. 6 is a cross-sectional view of said cam taken at 6-6 in Fig. 5;
Fig. 7 is a diagrammatic view of the steps of said cam;
Fig. 8 is a plan view of the internal support plate of said embodiment of shunt valve;
Fig. 9 is a cross-sectional view taken at 9-9 in Fig. 8;
Fig 10 is a plan view of the permanent-magnet disk of said embodiment of shunt valve, showing the ten pairs of poles on said disk;
Fig. 11 is a cross-sectional view of said disk taken at 11-11 in Fig. 10;
Fig. 12 is a diagrammatic view of said shunt valve in which the positions of the external adjusting electromagnets of an embodiment of apparatus constructed in accordance with the present invention are also shown (much smaller than actual size);
Fig. 13 is a diagrammatic view of an embodiment of apparatus constructed according to the present invention together with part of an assembly showing how they cooperate together for external adjustment of the shunt valve;
Fig. 14 is a diagrammatic view similar to Fig. 12 except that the rotor and cam of the shunt valve have been removed to show the magnetic polarization of the four stator elements;
Figs. 15 and 16 show the magnetic polarization of an alternative arrangement, where a one-piece stator element is used in the shunt valve;
Fig. 17 is a partial plan view, somewhat diagrammatic, of an alternative shunt valve wherein the stator elements each include an electrical coil; and
Fig. 18 is a diagrammatic view of the magnetic wheel and external adjusting electromagnets of another embodiment.

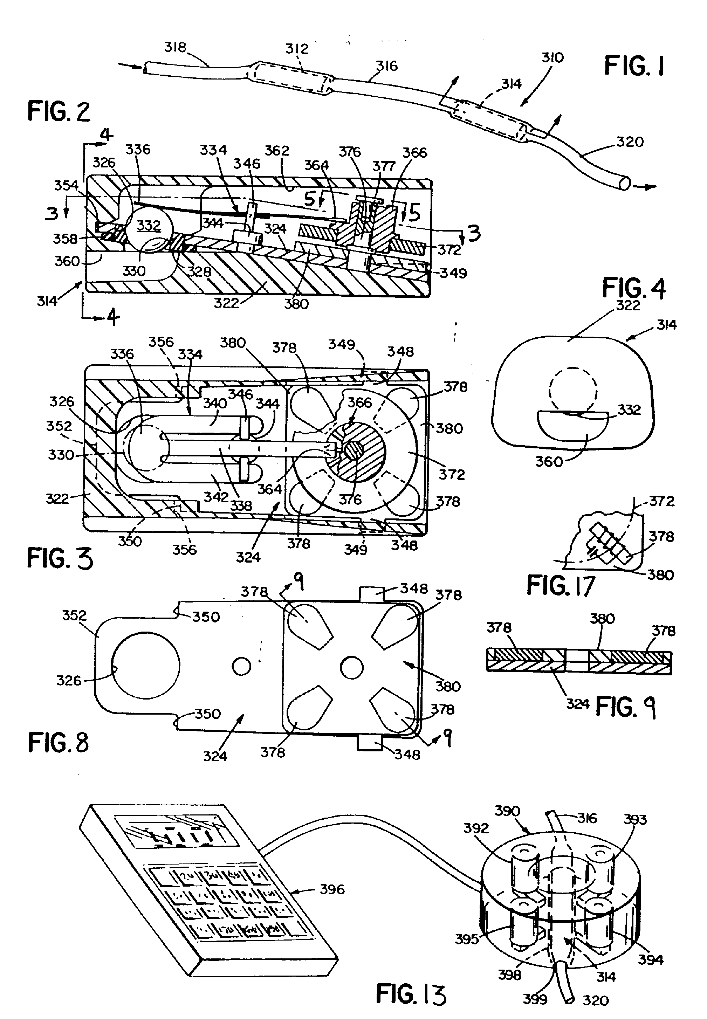

There is shown in Fig. 1 a shunt valve assembly 310 with two shunt valves 312, 314 separated by a pumping chamber 316. Cerebroventricular catheter 318 is connected to the inlet of the valve assembly, and drainage catheter 320, to the outlet. This assembly can be surgically implanted following well-known procedures.

A cross section through the downstream shunt valve 314 is shown in Fig. 2 The upstream valve 312 is preferably the same except that the adjustment mechanism is absent. (The tubular plastic covering shown tightly fitted around the valves; in Fig. 1 is not shown in the remaining figures.) Valve body 322 (injection molded from a surgically-implantable material such as polyethersulfone) has within its interior an inclined plate 324 made from a nonmagnetic material, such as titanium or stainless steel The plate 324 has circular aperture 326 in which is press fit a sapphire ring 328, with frustoconical surface 330 forming a valve seat for spherical ball 332 (highly-polished ruby).

Biasing the ball against the valve seat is spring 334 (single piece of stainless steel or another suitable material), shown in plan view in Fig. 3. The spring provides a low K factor to produce little change in working pressure with changes in flow (i.e., a flat flow-pressure curve). The spring has base 336 overlying ball 332, central arm 338 extending from the base to an adjustment mechanism, and two flanking arms 340, 342 extending from the base to a yoke 344. The yoke is press fit into a hole in plate 324 and tabs 346 extend over the tops of the flanking arms. The yoke is relieved in the center to provide room for the central arm to pass through. Notches (not shown) cut in the ends of flanking arms 340, 342 receive portions of the yoke, and secure the spring against longitudinal movement. The spring is secured against sideward movement by contact of the flanking arms with the vertical outside surfaces of the yoke.

Plate 324 is held tightly in place within valve body 322. The tight fit is achieved by sliding the plate into the valve body (in a direction from right to left in Fig. 2. Grooves 354, 356 at the upstream end of the valve body receive portions 350, 352 (Fig. 8) of the plate, and grooves 349 at the downstream end receive tabs 348 on the plate. The grooves extend generally horizontally rather than in the inclined direction followed by the plate, and thus the tabs 348 and portions 350, 352 tend to become tightly wedged into the grooves.

Grooves 354, 356 at the ball end of the valve body also serve to press plate 324 downwardly so as to squeeze it tightly against O-ring 358 (silicone rubber), which provides an internal seal to ensure that all flow through the valve is through the orifice formed between the ball 332 and the valve seat 330. Flow through the valve is from inlet cavity 360, past ball 332, and into outlet cavity 362.

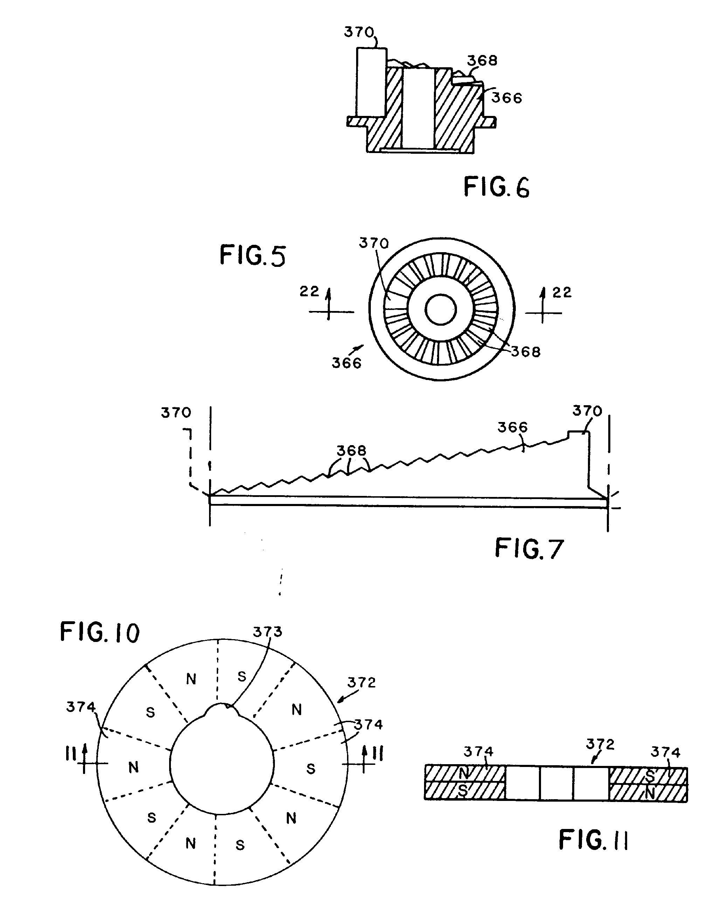

The preload of spring 334 against ball 332 is adjusted by using cam 366 (Delrin) to vary the vertical position (through a 0.75 mm range) of free end 364 of central arm 338. The spring preload establishes the pressure of the valve. The cam (best shown in Figs. 5, 6 and 7) has a circular staircase of eighteen steps, each being grooved so as to have a V-shape cross section. Free end 364 of arm 338 has a similar V-shape chosen to mate with the V-shape of steps 368. At each end of the staircase a barrier is provided by element 370. This confines rotation of the cam to slightly less than one revolution. The V-shape of steps 368 act as detents to keep the cam in precisely one of eighteen possible angular positions. That means that the vertical position of free end 364 of arm 338 is always at precisely one of eighteen-different values and, in turn, that the working pressure of the valve is always at one of eighteen possible levels.

Cam 366 is press fit into the central hole in rotor 372 (4 mm diameter), with a protrusion on the cam fitting into recess 373 in the rotor to assure accurate angular positioning. The cam-rotor unit rotates loosely on shaft 376, the base of which is press fit into plate 324. The unit is retained by retaining element 377 secured to the top of the shaft. The rotor is preferably made of platinum cobalt or samarium cobalt (which may be plated with platinum to improve corrosion resistance). The rotor has ten permanently magnetic poles 374 of alternate polarity (Figs. 10-11). At any one angular position, the pole exposed on the top surface of the disk is opposite that of the one exposed on the bottom surface.

Below rotor 372 there are fixed in place four stator elements 378 each made of a material that is magnetically soft and permeable, and that is resistant to corrosion in the presence of cerebrospinal fluid, which contains chlorides. Preferred materials include magnetic stainless steel alloys and alloys of nickel, iron, and molybdenum or cobalt As shown in Fig. 8, the stator elements are embedded in a plastic member 380, which is fixed to plate 324 by means of shaft 376. The stator elements are shaped so that the portion of each lying beneath the rotor matches the size of permanent magnets 374. The portions of the stator elements lying radially beyond the sized to match the area beneath the rotor so that the boundary between poles, when the stator is magnetized, is at the perimeter of the rotor.

### Operation

In operation the shunt valve assembly is surgically implanted in a patient following well-known procedures. Before implantation the pressure of adjustable valve 314 can be set to the desired level according to the circumstances of the case. For instance, it can be set approximately equal to the patient's pre-operative ventricular CSF pressure so that no immediate pressure change occurs as a result of the operation. After the patient has recovered from the trauma of the operation, the pressure is adjusted downwardly to the desired level. In the case of normal-pressure hydrocephalus, the pressure is lowered to a level sufficient to initiate shrinkage of the cerebral ventricle. Further adjustments in pressure can be made at subsequent times, as necessary. In the typical treatment of normal-pressure hydrocephalus, the pressure would be adjusted upwardly after sufficient shrinkage of the ventricle has occurred in order to stabilize ventricle size.

In children at the beginning of the treatment, the pressure should be lowered to a level inversely proportional to the ventricle size to reduce stress on the brain parenchyma (see Fig. 13 of Hakim et al., "The Physics of the Cranial Cavity", Surg. Neurol., Vol. 5, March 1976), and as the ventricle decreases in size the pressure of the valve should be increased, so when the ventricle attains normal size the intraventricular pressure is again normal, thereby avoiding development in the patient of a slit-ventricle condition. Also in cases of normal-pressure hydrocephalus, sometimes in spite of a low-pressure valve the patient does not improve and the ventricle size remains unchanged, making the surgeon think he is dealing with a case of brain atrophy. But by further changing the valve pressure to a lower one, the ventricle decreases in size and the patient immediately starts to improve. In elderly persons and in long standing cases of normal-pressure hydrocephalus, it has been found that the intraventricular pressure needs to be lowered more than in young people and in hydrocephalus of short duration.

Another advantage of the invention arises in the procedure for determining when an implanted shunt valve can safely be removed from a patient, i.e., determining whether the patient is still dependent on the valve for drainage of excess cerebrospinal fluid. The conventional technique for making that determination has been to temporarily pinch closed the tube downstream of the valve and observe the patient for symptoms (e.g., sight headache) indicative of valve dependency. In the absence of symptoms the valve can be removed. With the invention it is unnecessary to stop flow entirely. A safer procedure can be followed. Valve pressure is raised, slightly at first, more so later for confirmation, using the adjustment mechanism.

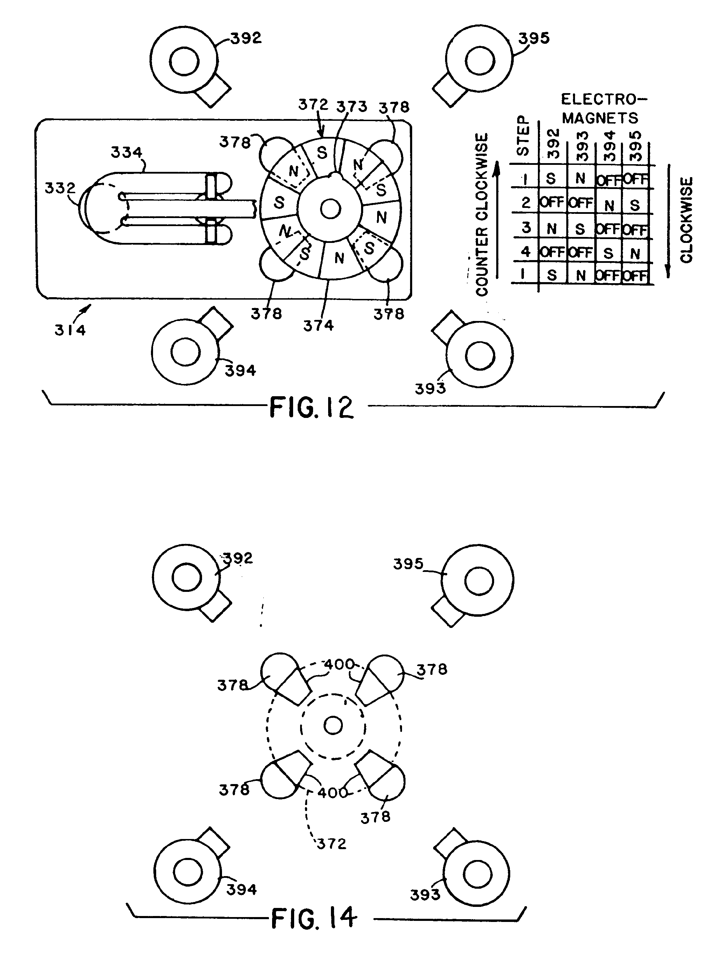

Valve pressure adjustments are made in the illustrated shunt valve by applying a pulsed magnetic field to the vicinity of the shunt valve as shown diagrammatically in Figs. 12-14. A valve adjustment element 390 is applied over adjustable valve 314 in the orientation shown. The adjustment element contains four electromagnets 392, 393, 394, and 395, which are separately controlled by an external control device, shown diagrammatically at 396. Adjustment element 390 has a marking (such as an arrow pointing in the direction of CSF flow) on its exterior to assure that it is applied to the valve in the correct orientation, and it has a groove 398 in its bottom surface sized to fit over the protrusion in the scalp, at the site of the implanted valve. The groove is narrowed at one end 399 to enable correct longitudinal alignment relative to the adjustable valve 314.

Control device 396 has input keys, which the operator uses to select one of 18 possible desired pressures (from 20 to 190 mm H₂0) and a pressure display.

Each of electromagnets 392, 393, 394, 395 can be energized to have either the north or south polarity facing the stator elements, or each can remain off altogether. Movement of rotor 372, in the desired direction and through the desired angle, is achieved by energizing the electromagnets in the sequence shown in the table in Fig. 12. for example, clockwise motion is achieved by first energizing electromagnets 392, 393 to south and north polarities, respectively, and leaving electromagnets 394, 395 off. In the next step electromagnets 392, 393 are left off, and electromagnets 394, 395 are energized to north and south polarities, respectively. The sequence repeats itself after the fourth step. Rotor 372 is shown in Fig. 12) in the position reached after the first step (the polarities of the rotor magnets are those on the bottom surface). If the magnetic field provided by the electromagnets is described by a vector pointing from the south to the north pole of the energized magnets, then it can be seen that the sequence prescribed for causing rotor 372 to rotate clockwise (down the table in Fig. 12) amounts to rotating the field vector in the counterclockwise direction (opposite that of the rotor), in 90° steps.

Electromagnets 392, 393, 394, 395 are positioned 90° apart and spaced equal radial distances from a central axis. When adjustment device 390 is installed properly over valve 314, the central axis of the electromagnets is coincident with the axis of rotation of rotor 372, and each electromagnet is aligned at the same angular position as one stator element 378. It is not, however, necessary that this alignment be exact. The invention is tolerant of alignment errors, which are unavoidable owing to the inability of the user to see rotor 372 or stator elements 378 and to the small size of those elements relative to the size of the external electromagnets.

The magnetic polarization induced in the stator elements 378 as the result of energizing the electromagnets is diagrammatically illustrated in Fig. 14) The two stator elements along the axis connecting the two energized electromagnets are polarized in the radial direction, so that the boundary between the poles lies roughly at the peripheral edge of disk rotor 372. The radially inner portions of these two stator elements, the portions lying beneath rotor 372, have the opposite polarity of the portions lying outside. By contrast, the stator elements along the other axis are polarized so that the boundary between poles lies along the radial direction. Both poles extend beneath the rotor 372. This pattern of polarization will result even if there is substantial error in the orientation of the electromagnets.

Movement of rotor 372 is influenced predominantly by the stator regions 400 (shown in dashed lines in Fig. 14) lying beneath the rotor, as it is those portions that are closest to the permanent magnets 374 of the rotor. Accordingly, the part of the stator elements with uniform polarity dominate over those with split polarity. This phenomenon could be emphasized by making the stator elements of a magnetically anisotropic material so that the magnetization induced by the external electromagnets is strongest along the radial axis of the corresponding stator elements.

The number of magnetic poles 374 is selected so that when one pair of radially opposite stator elements 378 is aligned with one pair of magnetic poles 374 (as are the upper left and lower right stator elements in Fig.12) the other two stator elements (the upper right and lower left in Fig. 12) are each staggered halfway between two of the poles 374. In operation, control device 396 energizes the electromagnets closest to the pair of stators staggered between two magnets, thereby causing the rotor to move through an angle corresponding to one half the width of a magnetic pole 374. In the preferred embodiment of shunt valve there are ten magnetic poles on each side of the disk, and thus twenty angular increments in one full revolution (i.e., each step is one twentieth of 360°, or 18°). Only eighteen of these increments are used, corresponding to the eighteen detented steps along the staircase surface of cam 368 (the other two increments are occupied by the detent wall 370 of the cam).

After a pressure is prescribed on control device 396, an enter key is pressed. That initiates a sequence of eighteen steps in the direction of lower pressure settings, counterclockwise rotation of rotor 372. This assures that the cam is returned to a position wherein spring arm 364 is at the lowest step on the cam staircase. If fewer than eighteen steps are actually needed to bring the cam to this position (as will most often be the case), the detent wall provided by element 370 of the cam prevents further rotation. After the eighteen-step resetting sequence is complete, the rotor is moved clockwise by the number of steps corresponding to the prescribed pressure.

Various variations can be made to the arrangement illustrated in the drawings.

A magnetically anisotropic material could be used for the stator elements, with the strongest axis of magnetization oriented along the radial direction. Such anisotropy could also be achieved mechanically by splitting each stator element along the radial direction into two or more segments.

A variable reluctance or hybrid rotor could replace permanent-magnet rotor 372.

Linear movements within an implanted device to adjust the popping pressure of a shunt valve could be achieved following the invention by providing a linearly-moving element as the rotor and by placing stator elements along the path of the linearly-moving rotor.

A rotor with fewer poles could replace the ten-pole rotor of the preferred embodiment of shunt valve, particularly where fine angular precision is not required (e.g., in a pump a simple two-pole rotor might suffice).

Electrical wire could be wrapped around the implanted stator elements forming a coil so that an electrical current is induced therein by the externally-pulsed magnetic field; the electrical current would in turn magnetize the stator elements if the coil circuit is closed by a resistor or a capacitor.

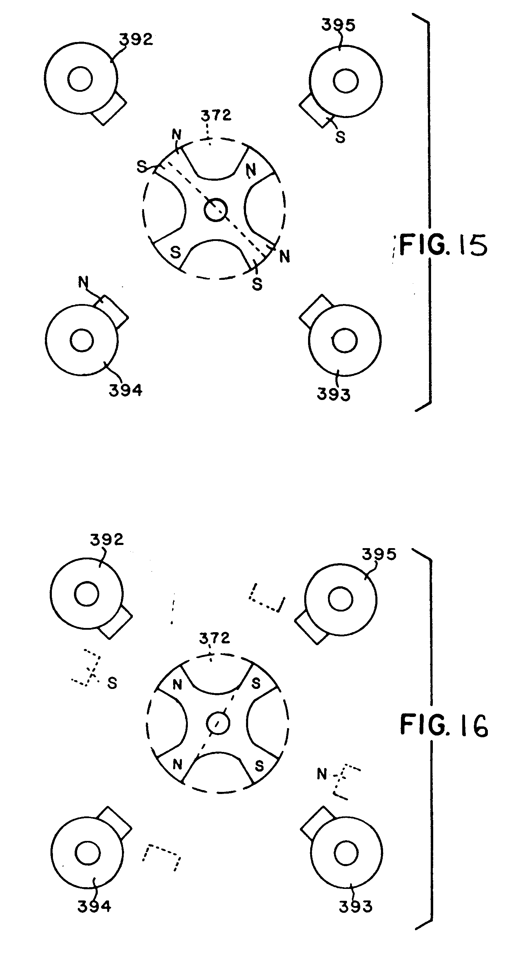

A single-piece stator element, e.g., with four lobes as shown in Fig. 15, could replace the stator elements of the preferred embodiment of shunt valve. An advantage of using a single-piece stator element is that the stator can lie entirely inside of the outer perimeter of the rotor, and thus provide for a more compact implanted unit. This is because, when magnetized, as shown in Fig. 15, the dominating lobes are all of one polarity, rather than split radially into two polarity regions as in the preferred embodiment. Thus the entire lobe, not just the inner half, can be influential in moving the rotor. The disadvantages of the single-piece stator is its lower tolerance to errors in alignment of the external magnetic field, The reduced tolerance for misalignment can be understood by reference to Fig. 16), in which the external field has been rotated sufficiently to cause the lobes that were split into two poles to now be entirely within the region of one pole. As a result all four lobes have nearly equal influence on the rotor, and it is not possible to move the rotor.
A presently even more preferred embodiment of shunt valve is one having a rotor 372 with six poles rather than ten (as shown in the figures). Such a rotor provides twelve steps of 30° for one full revolution. Eleven of these can be used to provide eleven different pressure Settings (each differing by 15 mm H₂O) from 30 to 180 mm H₂O. An advantage of six poles-is that with the four-stator-element configuration (Fig. 12) greater torque is available using six rather than ten poles. This results because all four stator elements, when magnetized by the external field, generate a torque in the same direction. In the ten-pole embodiment (Fig. 12), this is not the case. The torque generated by the stator elements with split polarity (upper right and lower left in Fig. 12) is opposite, though weaker than, that generated by the stator elements with uniform polarity. It is instructive to note, however, that this difference between the six and ten-pole embodiments is just the opposite if a one-piece, four-lobed stator is used. In that case, the split polarity stator elements generate opposing torque with the six-pole rotor and not with the ten-pole one Also, if a ten-pole rotor is used with separate stator elements, torque can be increased by using anisotropic material for the stator elements, as that will increase the dominance of the uniform polarity stator elements over the split polarity ones.

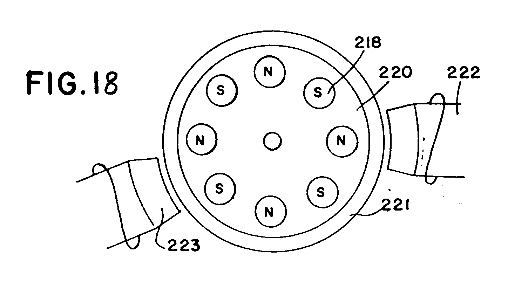

Another embodiment uses a very small stepper motor similar to the type used in electronic watches to rotate a spring adjustment screw for the popping pressure; the rotor of the stepping motor would reside in the valve, and the stator would be placed in the external adjustment element. (This contrasts with the embodiment of Figs. 1-17, wherein part of the stator resides in the valve). Such an arrangement is shown in Fig. 18. A plurality of alternate polarity permanent magnets 218 are formed on disk 220 (samarium-cobalt); one pole of each magnet 218 is on the top surface of the disk, the other pole is on the bottom surface. The disk 220 is attached to a wheel 221, to form the rotor portion of the stepper motor. External electromagnets 222, 223 are positioned as shown in Fig. 18 with respect to disk 220; when one external electromagnet 222 is aligned with one of magnets 218, the other external electromagnet 223 is staggered halfway between two of magnets 218. In operation, electromagnets 222, 223 are pulsed alternatively, so that the electromagnet pulsed is the one staggered between two of magnets 218. The direction of rotation of the disk 220 will be determined by the polarity of this magnet. This embodiment generally requires a large rotor and accurate positioning of the external stator.

## Claims

1. A method of indirect external adjustment of the popping pressure of a shunt valve of the type having one or more members adapted to move under the influence of a magnetic field, the method being characterised in comprising the steps of: positioning an adjustment device in proximity to said valve, said adjustment device including a plurality of electromagnets; and selectively energizing said electromagnets through a sequence of steps in which different ones of said electromagnets are energized at successive steps; the sequence of energization and the orientation and strength of said electromagnets being such that said member(s) is (are) caused to move in response to said sequence of energization and to thereby adjust said popping pressure.

2. A method according to Claim 1, further characterised in that said step of selectively energizing said electromagnets through a sequence includes changing the polarity (north or south) of said electromagnets from one step in the sequence to another.

3. A method according to Claim 2, further characterised in that at least one of said electromagnets has the reverse polarity of at least one other of said electromagnets during said sequence.

4. A method according to any preceding claim, further characterised in that there is a plurality of said members in said valve positioned around a first axis, in that said electromagnets are positioned around a second axis in said adjustment device, and in that said adjustment device is adapted to be positioned with respect to said valve so that said first and second axes are approximately coaxial.

5. A method according to Claim 4, further characterised in that there are four said electromagnets spaced 90° apart around said axis and in that said sequence consists of that shown in the table of Fig. 27.

6. A method according to any preceding claim, further characterised in that said electromagnets are energized to deliver a pulsed magnetic field, with each pulse corresponding to one step in said sequence, and in that said member(s) of said valve go(es) through an incremental movement for each pulse in said magnetic field.

7. A method according to Claim 4, further characterised in that said sequence is selected to cause the field vector of the magnetic field generated by said electromagnets to rotate about said second axis through an incremental angular movement at each step in said sequence, and said sequence and the strength and orientation of said electromagnets are selected so that said members are caused to rotate in the same direction about said first axis.

8. A method according to any preceding claim, further characterised in that a control panel with input keys for specifying popping pressure is provided, and keys corresponding to a desired popping pressure are depressed to initiate said sequence.

9. A method according to Claim 1, further characterised in that each step in said sequence causes an incremental movement of said member(s) and a corresponding incremental change in said popping pressure.

10. A method according to Claim 9, further characterised in that said sequence comprises a first sequence long enough to move said member(s) to a known reference position (e.g., the lowest pressure setting) from any possible starting position and a second sequence of length selected to move said member (s) through the number of increments corresponding to the desired popping pressure.

11. Apparatus for externally programming a shunt valve whereby indirectly and externally to adjust its popping pressure, said shunt valve being of the type having one or more members adapted to move under the influence of a magnetic field, said apparatus being characterised in comprising: a housing with a plurality of electromagnets supported on or within said housing; and means electrically connected to said electromagnets and adapted for selectively energizing said electromagnets through a sequence of steps in which different ones of said electromagnets are energized at successive steps, the sequence of energization and the orientation and strength of said electromagnets being selected such that said member(s) in said valve is (are) caused to move in response to said sequence of energization to thereby adjust said popping pressure.

12. Apparatus according to Claim 11, further characterised in that said energising means is adapted to energize said electromagnets through a sequence including changing the polarity (north or south) of said electromagnets from one step in the sequence to another.

13. Apparatus according to Claim 12, further characterised in that said sequence is selected such that at least one of said electromagnets has the reverse polarity of at least one other of said electromagnets during said sequence.

14. Apparatus according to any of Claims 11 to 13, wherein there is a plurality of said members in said valve positioned around a first axis, the apparatus being further characterised in that said electromagnets are positioned around an axis in said adjustment device, and in that said case is shaped to facilitate orienting it so that said first and second axes are approximately coaxial.

15. Apparatus according to Claim 14, further characterised in that there are four said electromagnets spaced 90° apart around said second axis and in that said sequence consists of that shown in the table of Fig. 27.

16. Apparatus according to Claim 11, further characterised in that said energizing means includes means adapted to generate a pulsed magnetic field, with each pulse corresponding to one step in said sequence, so that said member(s) of said valve is (are) operatively caused to go through an incremental movement for each pulse in said magnetic field.

17. Apparatus according to Claim 14, further characterised in that said sequence is selected to cause the field vector of the magnetic field operatively generated by said electromagnets to rotate about said second axis through an incremental angular movement at each step in said sequence, and in that said sequence and the strength and orientation of said electromagnets are selected such that said members are caused to rotate in the same direction about said first axis.

18. Apparatus according to any of Claims 11 to 17, further characterised in comprising a control panel with input keys adapted for specifying a desired popping pressure, and means adapted for operatively translating activation of said input keys to electrical signals for controlling said electromagnets.

19. Apparatus according to Claim 11, further characterised in that each step in said sequence is adapted to cause an incremental movement of said member(s) and a corresponding incremental change in said popping pressure.

20. Apparatus according to Claim 19, further characterised in that said sequence is selected to comprise a first sequence long enough to move said member(s) to a known reference position (e.g., the lowest pressure setting) from any possible starting position and a second sequence of length selected to move said member(s) through a number of increments corresponding to a desired popping pressure.

## Patentansprüche

1. Verfahren zur indirekten externen Einstellung des Öffnungsdruckes eines Nebenschluß-Ventiles (shunt) des Typs, das ein oder mehrere Teile besitzt, die unter dem Einfluß eines magnetischen Feldes bewegbar sind, wobei das Verfahren durch folgende Schritte
gekennzeichnet ist:
- Positionieren einer Einstellvorrichtung in der Nähe des genannten Ventiles, wobei die Einstellvorrichtung eine Vielzahl von Elektromagneten einschließt,
- selektives Erregen der Elektromagnete mittels einer Sequenz, bei der verschiedene der genannten Elektromagnete in sukzessiven Schritten erregt werden,
- die Sequenz der Erregung, der Orientierung und der Magnetfeldstärke der genannten Elektromagnete ist dabei derart, daß die Teile sich entsprechend der Erregungssequenz bewegen und dabei den Öffnungsdruck adjustieren.

2. Verfahren nach Anspruch 1, dadurch gekennzeichnet, daß der Schritt der selektiven,
sequentiellen Erregung der genannten Elektromagnete im Rahmen einer Schrittfolge die Änderung der Polarität (Nord oder Süd) besagter Elektromagnete umfaßt.

3. Verfahren nach Anspruch 2, dadurch gekennzeichnet, daß wenigstens einer der genannten Elektromagnete gegenüber wenigstens einem anderen der genannten Elektromagnete die umgekehrte Polarität während der vorgenannten Sequenz aufweist.

4. Verfahren nach einem der vorgehenden Ansprüche, dadurch gekennzeichnet, daß
- eine Vielzahl derartiger Teile in dem genannten Ventil vorhanden ist, die um eine erste Achse positioniert sind,
- und dadurch, daß die Elektromagnete um eine zweite Achse in der Einstellvorrichtung positioniert sind,
- und daß die genannte Einstellvorrichtung in eine Position in Bezug auf das genannte Ventil positionierbar ist, so daß die genannte erste und zweite Achse annähernd koaxial liegen.

5. Verfahren nach Anspruch 4, dadurch gekennzeichnet, daß vier derartige Elektromagnete vorhanden sind, die in einer Winkelstellung in Abständen von 90° um die genannte Achse angeordnet sind und daß die Sequenz derjenigen der Tabelle gemäß Fig. 27 entspricht.

6. Verfahren nach einem der vorhergehenden Ansprüche, dadurch gekennzeichnet, daß die Elektromagnete derartig erregt werden, daß sie ein gepulstes Magnetfeld liefern, wobei jeder Puls mit einem Schritt in vorgenannter Sequenz korrespondiert, und dadurch, daß die Teile (der Teil) des genannten Ventils für jeden Puls durch eine inkrementale Bewegung in dem genannten Magnetfeld gehen (geht).

7. Verfahren nach Anspruch 4, dadurch gekennzeichnet, daß die genannte Sequenz dahingehend gewählt ist, daß der durch die genannten Elektromagnete erzeugte Feldvektor des magnetischen Feldes um die zweite Achse durch inkrementale Winkelbewegung bei jedem Sequenzschritt zur Rotation gebracht wird, und daß die Sequenz und die Stärke und Orientierung der genannten Elektromagnete so ausgewählt sind, daß die genannten Teile in derselben Richtung um die erste Achse rotieren.

8. Verfahren nach einem der vorhergehenden Ansprüche, dadurch gekennzeichnet, daß ein Steuerungspanel mit Eingabetasten vorgesehen ist, um den Öffnungsdruck zu spezifizieren, und daß Tasten drückbar sind, die einem gewünschten Öffnungsdruck entsprechen, um die genannte Sequenz einzuleiten.

9. Verfahren nach Anspruch 1, dadurch gekennzeichnet, daß jeder Schritt in der genannten Sequenz eine inkrementale Bewegung der Teile und eine korrespondierende inkrementale Änderung bei dem genannten Öffnungsdruck bewirkt.

10. Verfahren nach Anspruch 9, dadurch gekennzeichnet, daß die Sequenz umfaßt:
- eine erste Sequenz, die lang genug ist, um die genannten Teile in eine bekannte Referenz-Position (z.B. dem niedrigsten Druck entsprechend) von einer beliebigen Startposition aus einzustellen,
- und eine zweite Sequenz, die entsprechend lang ist, um die Teile durch die Anzahl von Inkrementen zu bewegen, die dem gewünschten Öffnungsdruck entspricht.

11. Vorrichtung für eine externe Programmierung eines Nebenschluß-Ventiles (shunt), mit dem indirekt und von außen her dessen Öffnungsdruck adjustbar ist, wobei das Ventil ein oder mehrere Teile besitzt, die unter dem Einfluß eines magnetischen Feldes bewegbar sind, wobei die genannte Vorrichtung dadurch gekennzeichnet ist, daß sie folgende Teile umfaßt:
- ein Gehäuse mit einer Vielzahl von Elektromagneten, die am oder innerhalb des Gehäuses abgestützt sind;
- Mittel, die elektrisch mit den genannten Elektromagneten verbunden sind und geeignet sind, die genannten Elektromagnete selektiv im Rahmen einer schrittsequenz zu erregen, in deren Folge einzelne der genannten Elektromagnete in sukzessiven Schritten erregt werden, wobei die Erregungssequenz und die Orientierung und Stärke der genannten Elektromagnete dabei so gewählt sind, daß die Teile in dem genannten Ventil angeregt werden, sich entsprechend der Erregungssequenz zu bewegen und dabei den Öffnungsdruck zu adjustieren.

12. Vorrichtung nach Anspruch 11, dadurch gekennzeichnet, daß die Erregungsmittel, die die Elektromagnete erregen, so ausgelegt sind, daß sie diese im Rahmen einer Sequenz erregen, die die Änderung der Polarität
(Nord oder Süd) der genannten Elektromagnete in einer Sequenzfolge umfassen.

13. Vorrichtung nach Anspruch 12, dadurch gekennzeichnet, daß die Sequenz derart ausgewählt ist, daß wenigstens einer der genannten Elektromagnete gegenüber wenigstens einem der anderen Elektromagnete die umgekehrte Polarität während der Sequenz aufweist.

14. Vorrichtung nach Anspruch 11 bis 13, bei der eine Vielzahl derartiger Teile in dem genannten Ventil vorhanden sind, die um die erste Achse positioniert sind, dadurch gekennzeichnet daß die Elektromagnete um eine Achse in der Einstellvorrichtung positioniert sind, und daß diese in diesem Fall so geformt sind, daß sie die Orientierung zur Koaxialität der ersten und zweiten Achse erleichtert.

15. Vorrichtung nach Anspruch 14, dadurch gekennzeichnet, daß vier derartige Elektromagnete vorhanden sind, die in einer Winkelstellung in Abständen von 90° um die genannte Achse angeordnet sind und daß die Sequenz derjenigen der Tabelle gemäß Fig. 27 entspricht.

16. Vorrichtung nach Anspruch 11, dadurch gekennzeichnet, daß die Erregungsmittel Mittel umfassen, die ein gepulstes Magnetfeld liefern, wobei jeder Puls mit einem Schritt in vorgenannter Sequenz korrespondiert, so daß die Teile (der Teil) des genannten Ventils im Betrieb für jeden Puls durch eine inkrementale Bewegung in dem genannten Magnetfeld gehen (geht).

17. Vorrichtung nach Anspruch 14, dadurch gekennzeichnet, daß die genannte Sequenz dahingehend gewählt ist, daß der durch die genannten Elektromagnete im Betrieb erzeugte Feldvektor des magnetischen Feldes um die zweite Achse durch inkrementale Winkelbewegung bei jedem Sequenzschritt zur Rotation gebracht wird, und daß die Sequenz und die Stärke und Orientierung der genannten Elektromagnete so ausgewählt sind, daß die genannten Teile in derselben Richtung um die erste Achse rotieren.

18. Vorrichtung nach einem der vorhergehenden Ansprüche 11 bis 17, gekennzeichnet durch ein Steuerungspanel mit Eingabetasten, um einen Öffnungsdruck zu spezifizieren, und durch Mittel, die im Betrieb die Aktivierung der Eingabetasten in elektrische Signale zur Steuerung der genannten Elektromagnete übersetzen.

19. Vorrichtung nach Anspruch 11, dadurch gekennzeichnet, daß jeder Schritt in der genannten Sequenz eine inkrementale Bewegung der Teile und eine korrespondierende inkrementale Änderung bei dem genannten Öffnungsdruck bewirkt.

20. Vorrichtung nach Anspruch 19, dadurch gekennzeichnet, daß die Sequenz so ausgewählt ist, daß sie umfaßt:
- eine erste Sequenz, die lang genug ist, um die genannten Teile in eine bekannte Referenz-Position (z.B. dem niedrigsten Druck entsprechend) von einer beliebigen Startposition aus einzustellen,
- und eine zweite Sequenz, die lang genug ist, um die Teile durch die Anzahl von Inkrementen zu bewegen, die dem gewünschten Öffnungsdruck entspricht.

## Revendications

1. Une méthode d'ajustement indirect externe de la pression de sortie d'une valve de dérivation du type ayant une ou plusieurs barres adaptées pour se déplacer sous l'induction d'un champ magnétique, caractérisée par les étapes suivantes : positionnement d'un dispositif d'ajustement à proximité de ladite valve, ce dispositif d'ajustement comprenant une pluralité d'électro-aimants; excitation sélective de ces électro-aimants par une suite de périodes au cours desquelles certains des différents électro-aimants sont traversés par un courant périodique ; la période d'excitation, l'orientation et la force desdits électro-aimants étant telles que la (les) barre(s) est (sont) entraînée(s) à se déplacer en réponse à cette séquence d'excitation et de ce fait ajuste(nt) la pression de sortie.

2. Méthode selon la Revendication 1 caractérisée en outre en ce que la période d'excitation sélective des électro-aimants lors d'une séquence comprend le changement de polarité (nord ou sud) de ces électro-aimants d'une période à l'autre.

3. Méthode selon la Revendication 2 caractérisée en outre en ce qu'au moins l'un des électro-aimants a, durant la séquence, la polarité inversée de l'un au moins des autres électro-aimants.

4. Méthode selon l'une quelconque des revendications précédentes, caractérisée en outre en ce qu'une pluralité de barres dans la valve sont positionnées autour d'un premier axe, en ce que les électro-aimants sont positionnés autour d'un second axe dans l'ajustement du dispositif, et en ce que cet ajustement est adapté pour être positionné par rapport à la valve de sorte que les premier et second axes soient approximativement coaxiaux.

5. Méthode selon la Revendication 4 caractérisée en outre en ce que les quatre électro-aimants sont espacés entre eux de 90° autour dudit axe, et en ce que la séquence permet la réalisation apparaissant sur le tableau de la Figure 27.

6. Méthode selon l'une quelconque des revendications précédentes caractérisée en outre en ce que les électro-aimants sont excités pour délivrer un champ magnétique pulsé, chaque pulsion correspondant à une période de cette séquence, et en ce que la (les) barre(s) de la valve est (sont) soumise(s) à un mouvement incrémentiel à chacune des pulsions du champ magnétique.

7. Méthode selon la Revendication 4, caractérisée en outre en ce que la séquence est choisie pour que le champ vectoriel du champ magnétique occasionné par les électro-aimants vienne à chaque période séquentielle tourner autour du second axe par un mouvement angulaire incrémentiel, cette séquence, la force et l'orientation desdits électro-aimants étant choisis pour que les barres soient entraînées à venir tourner dans le même sens autour du premier axe.

8. Méthode selon l'une quelconque des revendications précédentes, caractérisée en outre par un panneau de contrôle des clavettes de débit déterminant la pression de sortie, les clavettes correspondant à la pression de sortie désirée étant enfoncées pour amorcer la séquence.

9. Méthode selon la revendication 1, caractérisée en outre en ce que chaque période séquentielle produit un mouvement incrémentiel de la (des)dite(s) barre(s) et un changement incrémentiel correspondant dans la pression de sortie.

10. Méthode selon la revendication 9, caractérisée en outre en ce que la séquence comprend une première période assez longue pour actionner à partir de n'importe quelle position de départ la (les) barre(s) vers une position de référence connue (par exemple, la pression de fonctionnement la plus basse) , et une second période de longueur déterminée pour déplacer la (les) barre(s) au travers du champ d'incréments correspondant à la pression de sortie désirée.

11. Appareil destiné à la programmation externe d'une valve de dérivation grâce auquel la pression de sortie peut être ajustée indirectement de l'extérieur, cette valve de dérivation étant du type de valve ayant une ou plusieurs barre(s) adaptées pour se déplacer sous l'influence d'un champ magnétique, ledit appareil étant caractérisé par : un bâti abritant une pluralité d'électro-aimants maintenus sur ou à l'intérieur dudit bâti ; et des moyens connectés électriquement à ces électro-aimants et prévus pour exciter sélectivement ces électro-aimants à la faveur de périodes séquentielles au cours desquelles des électro-aimants sont excités à tour de rôle, la séquence d'excitation, d'orientation et de force desdits électro-aimants étant choisie de sorte que la (les) barre(s) à l'intérieur de ladite valve est (sont) amenée(s) à se déplacer en réponse à ladite séquence d'excitation, pour de cette façon ajuster la pression de sortie.

12. Appareil selon la Revendication 11, caractérisé en outre en ce que lesdits moyens d'excitation sont adaptés pour exciter lesdits électro-aimants durant une séquence comprenant un changement de polarité (nord ou sud) desdits électro-aimants d'une étape à l'autre.

13. Appareil selon la Revendication 12, caractérisé en outre en ce que la séquence est choisie de telle sorte que durant cette séquence au moins un des électro-aimants a la polarité inversée de l'un au moins des autres électro-aimants.

14. Appareil selon l'une quelconque des Revendications 11 à 13, dans lequel il y a pluralité de barres dans la valve positionnées autour d'un premier axe, l'appareil étant en outre caractérisé en ce que les électro-aimants sont positionnés autour d'un axe dans le dispositif d'ajustement, et en ce que le bâti est façonné de manière à faciliter son orientation pour que les premier et second axes soient approximativement coaxiaux.

15. Appareil selon la Revendication 14, caractérisé en outre en ce qu'il y a des électro-aimants espacés de 90° chacun autour du second axe et en ce que la séquence permet la réalisation montrée sur le tableau de la Figure 27.

16. Appareil selon la Revendication 11, caractérisé en outre en ce que les moyens d'excitation comprennent des moyens adaptés pour générer un champ magnétique pulsé, chaque pulsion correspondant à une étape de cette séquence, de sorte que la (les) barre(s) de la valve est (sont) de façon opérative mises en mouvement par les électro-aimants pour tourner à chaque période de la séquence autour du second axe selon un mouvement angulaire incrémentiel, et en ce que cette séquence, la force et l'orientation des électro-aimants sont choisis de telle manière que les barres sont amenées à tourner dans le même sens autour du premier axe.

17. Appareil selon la Revendication 14, caractérisé en outre en ce que la séquence est choisie pour permettre au vecteur du champ magnétique généré par les électro-aimants de tourner autour du second axe suivant un mouvement angulaire incrémentiel lors de chaque période séquentielle, et en ce que la séquence, la force et l'orientation des électro-aimants sont choisies de telle manière que les barres sont entraînées à tourner dans le même sens autour du premier axe.

18. Appareil selon l'une quelconque des Revendications 11 à 17, caractérisé en outre en ce qu'il comprend un tableau de contrôle avec des clavettes de débit prévues pour assurer une pression de sortie déterminée, et des moyens adaptés à la traduction opérationnelle de l'activation des clavettes en signaux électriques de contrôle des électro-aimants.

19. Appareil selon la Revendication 11, caractérisé en outre en ce que chaque période de la séquence est prévue pour permettre un mouvement incrémentiel de(s) barre(s) et un changement incrémentiel correspondant dans la pression de sortie.

20. Appareil selon la Revendication 19, caractérisé en outre en ce que ladite séquence est choisie pour comprendre une première séquence suffisamment longue pour déplacer la (les) barre(s) vers une position de référence (par exemple, la pression de fonctionnement la plus basse) à partir de n'importe quelle position de départ et une seconde séquence de longueur choisie pour déplacer la (les) barre(s) au travers d'un champ d'incréments correspondant à la pression de sortie désirée.
